(19) Europäisches Patentamt
European Patent Office
Office européen des brevets

(11) **EP 1 908 404 A1**

(12) **EUROPEAN PATENT APPLICATION**
published in accordance with Art. 153(4) EPC

(43) Date of publication:
**09.04.2008 Bulletin 2008/15**

(21) Application number: **06780990.5**

(22) Date of filing: **12.07.2006**

(51) Int Cl.:
*A61B 6/00* (2006.01)    *G06T 1/00* (2006.01)
*G06T 7/60* (2006.01)    *A61B 5/055* (2006.01)

(86) International application number:
**PCT/JP2006/313820**

(87) International publication number:
**WO 2007/013300 (01.02.2007 Gazette 2007/05)**

(84) Designated Contracting States:
**AT BE BG CH CY CZ DE DK EE ES FI FR GB GR
HU IE IS IT LI LT LU LV MC NL PL PT RO SE SI
SK TR**

(30) Priority: **27.07.2005 JP 2005217223**

(71) Applicant: **Konica Minolta Medical & Graphic, Inc.
Hino-shi,
Tokyo 191-8511 (JP)**

(72) Inventor: **KOBAYASHI, Tsuyoshi
Konica Minolta
Shinjuku-ku,
Tokyo 1630512 (JP)**

(74) Representative: **Henkel, Feiler & Hänzel
Patentanwälte
Maximiliansplatz 21
80333 München (DE)**

(54) **ABNORMAL SHADE CANDIDATE DETECTION METHOD AND ABNORMAL SHADE CANDIDATE DETECTION DEVICE**

(57)    It is possible to improve processing efficiency and detection accuracy during detection of an abnormal shade candidate. In an image processing device (2), an inputted breast image is reduced and subjected to a smoothing processing by using a first and a second smoothing filter so as to extract a detection object region of an abnormal shade candidate. After this, in the extracted region, an abnormal shade candidate is detected by using a curved filter and an abnormal shade candidate region is detected. The detection result is displayed.

## FIG. 1

**Description**

**FIELD OF THE INVENTION**

The present invention is relates to an abnormal shadow candidate detecting method and an abnormal shadow candidate detecting apparatus, each of which is to be employed for detecting an abnormal shadow candidate from a medical image.

**TECHNICAL BACKGROUND**

In the field of medical diagnosis, the digitalization of the medical images has been put into practice to such an extent that the medical images are displayed on the monitor, based on the medical image data generated by the CR (Computed Radiography) or the like, so that the doctor can make a diagnosis for a certain lesion part included in the medical images displayed on the monitor by observing the current status of the lesion and its variations with time.

To alleviate such the burden of the abovementioned medical image inspection practice to be conducted by the doctor, conventionally in the field of medical diagnosis, there has been developed the abnormal shadow candidate detecting apparatus called the Computer-Aided Diagnosis Apparatus (hereinafter, referred to as the CAD, for simplicity) that automatically detects a shadow of a certain lesion part to be emerged on the concerned medical image as an abnormal shadow candidate.

Since most of such the shadows of the lesion parts have characterized density distributions, the CAD detects such an image area that is possibly estimated as a lesion part on the basis of such the density characteristics, as an abnormal shadow candidate area.

With respect to the CAD mentioned in the above, various kinds of detecting algorism, corresponding to the kinds of lesions to be detected, have been developed. So far, the methodology employing the iris filter, the other methodology utilizing the characterizing amount of the curvature, etc., have been proposed as the optimum algorisms for detecting the tumor shadows. Further, the methodology employing the morphological filter, etc., has been proposed as the optimum algorisms for detecting shadows of micro calcified clusters (for instance, refer to in Patent Documents 1 and 2).

[Patent Document 1]
Tokkai 2002-112986 (Japanese Non-Examined Patent Publication)
[Patent Document 2]
Tokkai 2001-346787 (Japanese Non-Examined Patent Publication)

**DISCLOSURE OF THE INVENTION**

**SUBJECT TO BE SOLVED BY THE INVENTION**

According to Patent Document 1, when plural kinds of abnormal shadow detecting objects exist in the same radiographing portion, by making it possible for the doctor to select a single or a plurality of abnormal shadow candidate detecting algorism(s) corresponding to the detecting object(s), it becomes possible to shorten the processing time. However, since the arithmetic calculation processing are performed one pixel by next pixel all over the concerned medical image by employing the single or the plurality of abnormal shadow candidate detecting algorism (s) selected by the doctor, there has been such a problem that it still takes much processing time to search over the whole image, though the processing time can be reduced to a shorter time, compared to such the case that all of the possible abnormal shadow candidate detecting algorisms, including unnecessary algorisms, are employed for the arithmetic calculation processing. Further, there has been still another problem that either a noisy area or a normal tissue area, other than the lesion part, is erroneously detected as the abnormal shadow candidate.

Further, Patent Document 2 sets forth such the disclosure that, when the area at which the abnormal shadow candidate would exist is predictable for the doctor to a certain extent, or when the abnormal shadow candidate has been already detected in the other medical image, such as the medical image captured in the past, etc., the operation for detecting the abnormal shadow candidate is performed by only designating the area concerned. However, there has been a problem that another abnormal shadow residing in an area, other than the area being predictable for the doctor or detected in the past, cannot be detected, even if such the abnormal shadow exist outside the detection objective area. Further, since the arithmetic calculation processing are performed one pixel by next pixel within the designated area, there has been a possibility that either a noisy area or a normal tissue area, other than the lesion part, is erroneously detected as the abnormal shadow candidate, even if the area to be searched is limited. Still further, it has been impossible to classify the abnormal shadow candidates, detected in the above, into various sizes.

Still further, according to the methodology utilizing the characterizing amount of the curvature, in which the tumor shadow is regarded as the detecting object, the image area having a certain predetermined pattern, such as a convex shape, etc., can be sensitively detected by utilizing the localized information. However, since the predetermined areas should be sequentially established one by one over the whole image area, so as to calculate the curvature for every one of the predetermined areas, only such an abnormal shadow candidate that can be totally accommodated within the predetermined area, curvature of which is calculated, can be detected. Although it may be considered such a countermeasure that the detecting operation is performed while adaptively changing the range of area corresponding to the dimension of the abnormal shadow, the processing time would drastically increase, since the same processing should be repeatedly performed for many times while changing the range of area.

Yet further, since the detecting operation is performed by using such the localized information as the curvature in the detection processing according to the curvature, even the image area of the normal tissue is possibly detected as the area of the abnormal shadow candidate as far as the concerned image area has the predetermined pattern. As a result, sometimes, a number of erroneous positive candidates included in the detected result has increased.

The subject of the present invention is to improve a processing efficiency and a detecting accuracy at the time of detecting the abnormal shadow candidate.

## MEANS FOR SOLVING THE SUBJECT

The invention, recited in claim 1, is characterized in that, in an abnormal shadow candidate detecting method, the method comprises:

> a first processing process for applying a first smoothing filter processing to medical image inputted;
> a second processing process for applying a second smoothing filter processing to processed image to which the first smoothing filter processing is applied;
> an extraction processing process for extracting a specific area, from which an abnormal shadow candidate is to be detected, from processed image to which the second smoothing filter processing is applied;
> a characteristic amount calculating process for calculating a characteristic amount representing a curved shape indicating a density distribution of the specific area extracted; and
> an abnormal shadow candidate detecting process for conducting a detection processing of the abnormal shadow candidate, based on the characteristic amount calculated.

The invention, recited in claim 2, is characterized in that, in the abnormal shadow candidate detecting method recited in claim 1, the method further comprises a process for reducing the medical image inputted; and
the first smoothing filter processing is applied to the reduced medical image inputted in the first processing process.

The invention, recited in claim 3, is characterized in that, in the abnormal shadow candidate detecting method recited in claim 1 or 2, a Shape Index is calculated as the characteristic amount, representing the curved surface indicating the density distribution, in the characteristic amount calculating process.

The invention, recited in claim 4, is characterized in that, in the abnormal shadow candidate detecting method recited in any one of claims 1 - 3, an abnormal shadow species to be established as an detection object in the abnormal shadow candidate detecting process is a tumor.

The invention, recited in claim 5, is characterized in that, in an abnormal shadow candidate detecting method, the method comprises:

> a process for setting a first smoothing filter corresponding to a first abnormal shadow size to be extracted;
> a process for setting a second smoothing filter corresponding to a second abnormal shadow size to be extracted;
> an extracting process for extracting a specific area having a desired dimension to detect an abnormal shadow candidate, by applying the first smoothing filter and the second smoothing filter to medical image inputted;
> a characteristic amount calculating process for calculating a characteristic amount representing a curved shape indicating a density distribution of the specific area; and
> an abnormal shadow candidate detecting process for conducting a detection processing of the abnormal shadow candidate, based on the characteristic amount calculated.

The invention, recited in claim 6, is characterized in that, in an abnormal shadow candidate detecting apparatus, the apparatus comprises:

> a first smoothing processing means for applying a first smoothing filter processing to medical image inputted;
> a second smoothing processing means for applying a second smoothing filter processing to processed image to which the first smoothing filter processing is applied;

an extracting means for extracting a specific area, from which an abnormal shadow candidate is to be detected, from processed image to which the second smoothing filter processing is applied;
a characteristic amount calculating means for calculating a characteristic amount representing a curved shape indicating a density distribution of the specific area extracted; and
an abnormal shadow candidate detecting means for conducting a detection processing of the abnormal shadow candidate, based on the characteristic amount calculated.

The invention, recited in claim 7, is characterized in that, in the abnormal shadow candidate detecting apparatus recited in claim 6, the apparatus further comprises a reduction processing means for reducing the medical image inputted; and
the first smoothing processing means applies the first smoothing filter processing to the reduced medical image inputted.
The invention, recited in claim 8, is characterized in that, in the abnormal shadow candidate detecting apparatus recited in claim 6 or 7, the characteristic amount calculating means calculates a Shape Index as the characteristic amount, representing the curved surface indicating the density distribution
The invention, recited in claim 9, is characterized in that, in the abnormal shadow candidate detecting apparatus recited in claims 6 - 8, an abnormal shadow species to be established as an detection object in the abnormal shadow candidate detecting process is a tumor.
The invention, recited in claim 10, is characterized in that, in an abnormal shadow candidate detecting apparatus, the apparatus comprises:

a first setting means for setting a first smoothing filter corresponding to a first abnormal shadow size to be extracted;
a second setting means for setting a second smoothing filter corresponding to a second abnormal shadow size to be extracted;
an extracting means for extracting a specific area having a desired dimension to detect an abnormal shadow candidate, by applying the first smoothing filter and the second smoothing filter to medical image inputted;
a characteristic amount calculating means for calculating a characteristic amount representing a curved shape indicating a density distribution of the specific area; and
an abnormal shadow candidate detecting means for conducting a detection processing of the abnormal shadow candidate, based on the characteristic amount calculated.

## EFFECT OF THE INVENTION

According to the inventions recited in any one of claims 1, 3, 4, 7, 8 and 9, it becomes possible to exclude in advance such an area that has a size to be regarded as a false positive, judging from expected dimensions of the corresponding tumor concerned, from the detection objects. Further, by applying the detection processing of the abnormal shadow candidate, which employs the characteristic amount of the curvature so as to sensitively detect an area having a predetermined pattern, only to the extracted area, it becomes possible to reduce a number of false positive candidates. In other words, when applying such the detection processing as the curvature that has a high detection sensitivity, by conducting the area extraction processing using the smoothing filter preceding to the detection processing, it becomes possible to obtain a synergistic effect combining the reduction of the processing time and the improvement of the detecting accuracy with each other.
According to the inventions recited in claim 2 or 6, it becomes possible to reduce the processing time required for the medical image processing, such as the smoothing filter processing, the detection processing of the abnormal shadow candidate, etc.
According to the inventions recited in claim 5 or 10, by applying the first and the second smoothing filter, which are different from each other in the size of abnormal shadow to be processed, it becomes possible to change the detection objective area to be extracted, corresponding to the dimensions of the abnormal shadow area to be detected. Accordingly, it becomes possible not only to extract an area corresponding to the size of the abnormal shadow species of the detection object, but also to reduce a number of false positive candidates, by applying the detection processing of the abnormal shadow candidate, which employs the characteristic amount of the curvature so as to sensitively detect an area having a predetermined pattern, only to the extracted area. In other words, when applying such the detection processing as the curvature that has a high detection sensitivity, by conducting the area extraction processing using the smoothing filter preceding to the detection processing, it becomes possible to obtain a synergistic effect combining the reduction of the processing time and the improvement of the detecting accuracy with each other. Further, since it becomes possible to extract the detection objective areas while classifying them into various sizes of the abnormal shadow candidates to be detected, it becomes possible to conduct an operation for detecting the abnormal shadow candidate according to a plurality of detecting models.

**BRIEF DESCRIPTION OF THE DRAWINGS**

Fig. 1 shows a system configuration of a medical imaging system 100 embodied in the present invention.

Fig. 2 shows a internal configuration of an image processing apparatus shown in Fig. 1.

Fig. 3 shows an explanatory flowchart for explaining a lesion detection processing to be conducted by an image processing apparatus.

Fig. 4 shows an explanatory flowchart for explaining an area extraction processing shown in Fig. 3.

Fig. 5(a), Fig. 5(b), Fig. 5(c) and Fig. 5(a) show graphs schematically indicating image data to be generated in steps of an area extraction processing, respectively.

Fig. 6 shows an explanatory schematic diagram for explaining a first smoothing filter.

Fig. 7 shows an explanatory schematic diagram for explaining a second smoothing filter.

Fig. 8 shows a schematic diagram indicating a processing procedure when extracting detection objective areas while classifying them into sizes by employing first and second smoothing filters.

Fig. 9(a) shows an explanatory schematic diagram for explaining a curved surface representing density distribution, while Fig. 9(b) shows an explanatory schematic diagram for explaining a rotation of normal plane around the normal line of a pixel of interest (attention pixel).

Fig. 10 shows a curved surface representing density distribution in a digital image.

Fig. 11 shows an explanatory graph for explaining a method for calculating a curvature of a curved line, by employing the least square method to approximate a curved line indicating density distribution to a circle.

Fig. 12 shows various kinds of curved shapes corresponding to values of Shape Indexes.

Fig. 13 shows an explanatory schematic diagram for explaining an operation for scanning a curvature filter within a range of a processing objective image.

Fig. 14 shows an explanatory flowchart for explaining a detection processing of the abnormal shadow candidate, employing a curvature filter.

**EXPLANATION OF THE NOTATIONS**

100　　a medical imaging system
1　　an image data creating apparatus
2　　an image processing apparatus
21　　a CPU
22　　an operating section
23　　a display section
24　　a RAM
25　　a storage section
26　　a communication controlling section
27　　a bus

**BEST MODE FOR IMPLEMENTING THE INVENTION**

Referring to the case of detecting a candidate of tumor shadow from a medical image acquired by radiographing a breast (a breast image), an abnormal shadow candidate detecting method and an abnormal shadow candidate detecting apparatus, embodied in the present invention, will be detailed in the following.

Initially, the configuration of the present embodiment will be detailed in the following.

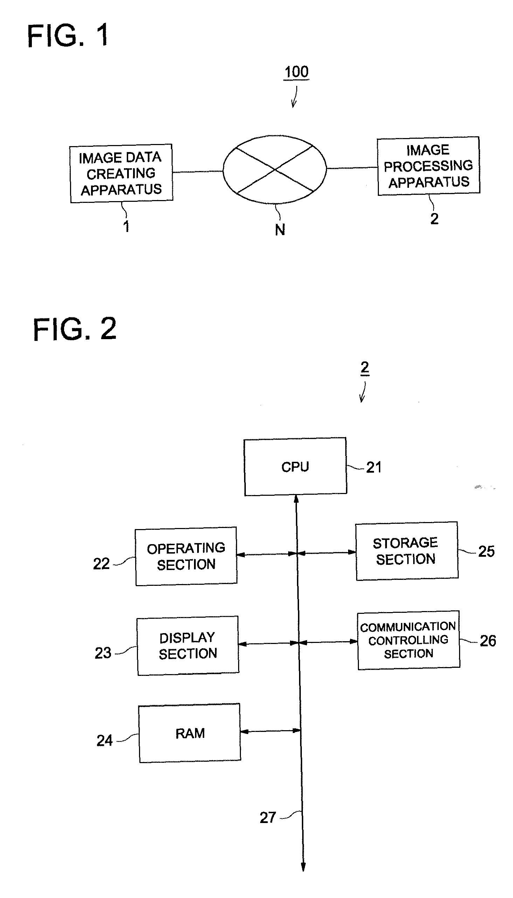

Fig. 1 shows a system configuration of a medical imaging system 100 embodied in the present invention.

As shown in Fig. 1, the medical imaging system 100 is constituted by an image data creating apparatus 1, an image processing apparatus 2, etc., which are coupled to each other through a network N, so as to make it possible to bilaterally communicate with each other. Further, the network N is conformity with the DICOM (Digital Imaging and Communication in Medicine) standard.

In this connection, although the system in which the image data creating apparatus 1 and the image processing apparatus 2 are coupled to each other through the network N is exemplified in the present embodiment, the scope of the system is not limited to the above-exemplified system. A directly wired system configuration is also applicable in the present invention. Further, it is also applicable that the system is so constituted that a server for controlling and storing the image data representing the medical images created by the image data creating apparatus 1, a monitor for displaying the detected results of the abnormal shadow candidates and the processed images outputted by the image processing apparatus 2, a film outputting apparatus for outputting film images, etc., are further coupled to each other though the network N, in addition to the image data creating apparatus 1 and the image processing apparatus 2.

The image data creating apparatus 1 includes various kinds of modalities, such as a CR (Computed Radiography), a FPD (Flat Panel Detector), a CT (Computed Tomography), a MRI (Magnetic Resonance Imaging), etc., each of which captures a medical image of the human body and converts the captured image to digital image data, so as to generate medical image data. In the present embodiment to be detailed in the following, the image data creating apparatus 1 is assumed as such the apparatus that radiographs the breast, so as to generate the medical image data of the breast image.

The image processing apparatus 2 serves as an abnormal shadow candidate detecting apparatus that applies an abnormal shadow candidate detection processing to the medical image data transmitted from the image data creating apparatus 1.

Next, the internal configuration of the image processing apparatus 2 will be detailed in the following.

Fig. 2 shows a functional configuration of the image processing apparatus 2. As shown in Fig. 2, the image processing apparatus 2 is provided with a CPU (Central Processing Unit) 21, an operating section 22, a display section 23, a RAM (Random Access Memory) 24, a storage section 25, a communication controlling section 26, etc., which are coupled to each other through a bus 27.

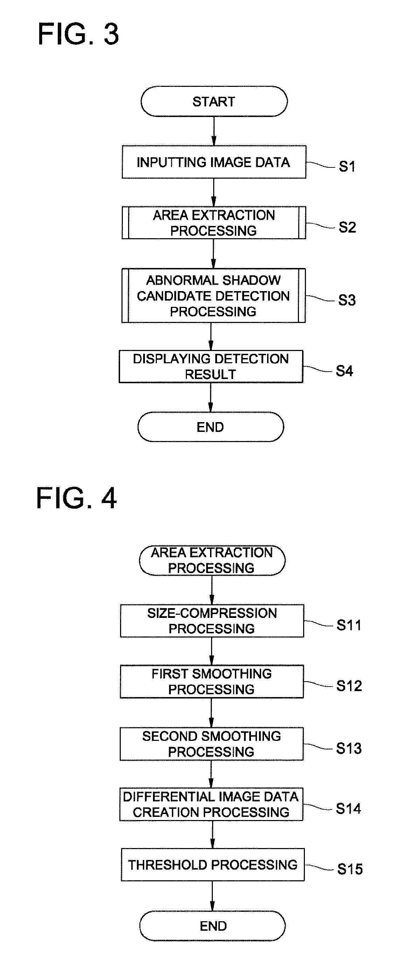

The CPU 21 reads out a system program stored in the storage section 25 and develops the system program into a working area created in the RAM 24, so as to control the sections concerned, according to the system program developed. Further, the CPU 21 also reads out various kinds of processing programs, such as a detection processing program, etc., which are stored in the storage section 25, and develops each of them into a corresponding working area created in the RAM 24, so as to implement various kinds of processing, such as a lesion detection processing detailed later (refer to Figs. 3, 4 and 14), etc.

The operating section 22 is provided with a keyboard, including a cursor key, ten keys, various kinds of function keys, etc., and a pointing device, such as a mouse, etc., so as to output the instruction signals, inputted by operating the keyboard and the mouse, to the CPU 21. Further, it is also applicable that the operating section 22 is constituted by a touch panel provided on a display screen of the display section 23, and in this configuration, the instruction signals inputted trough the touch panel are outputted to the CPU 21.

The display section 23 includes a display monitor, such as a LCD (Liquid Crystal Display), a CRT monitor, etc., in order to display various kinds of images, etc., according to the display command signals sent from the CPU 21.

The RAM 24 creates the working areas into which various kinds of programs executable by the CPU 21 and read out from the storage section 25, inputted or outputted data, various kinds of parameters, etc., which are implemented and controlled by the CPU 21 in various kinds of processing, are temporarily stored.

The storage section 25 is constituted by a HDD (Hard Disc Drive), a nonvolatile semiconductor memory, etc., so as to store the system program to be executed by the CPU 21, the various kinds of processing programs, such as the lesion detection processing program, etc., various kinds of data, etc., therein. The abovementioned various kinds of programs are stored in a mode of readable program codes, so that the CPU 21 can execute each of the abovementioned various kinds of programs as needed, according to the program code concerned.

Further, the storage section 25 is provided with a characteristic amount file into which a characteristic amount of a curved surface indicating a density distribution of the medical image (for instance, a Shape Index), which is calculated at the time of implementing the detection processing of the abnormal shadow candidate, is to be stored.

The communication controlling section 26 is provided with a LAN adaptor, a router, a TA (Terminal Adaptor), etc., so as to control the communications to be conducted between the apparatuses coupled to each other through the network N.

Next, the operations to be conducted in the present embodiment will be detailed in the following.

Fig. 3 shows a flowchart indicating the detection processing to be conducted by the CPU 21 of the image processing apparatus 2. The CPU 21 implements the abovementioned detection processing by executing the software processing in conjunction with the detection processing program stored in the storage section 25.

At first, medical image data D of a breast image, created by radiographing a breast in the image data creating apparatus 1, are inputted into the image data creating apparatus 1 through the communication controlling section 26, so as to store the medical image data D into the working area created in the RAM 24 (Step S1).

Successively, the CPU 21 applies an area extraction processing to the medical image data D inputted (Step S2). The area extraction processing is such a processing that extracts a detection objective area from the whole area represented by the medical image data D, corresponding to a size of the lesion part to be detected. For instance, the lesions including a tumor shadow, a micro calcified cluster, etc., can be cited as the major lesions to be made diagnosis by using the breast image. The tumor shadow is projected as a whitish and circular shadow, which could be recognized as a cluster being massive to a certain extent and exhibits such a density distribution that is near to the Gaussian distribution, on the breast image. On the other hand, the micro calcified cluster is projected as a small and white shadow, which exhibits such a density distribution that is substantially a circular conic structure, on the breast image. In other words, the depression of density value (pixel value) can be recognized at the lesion part in the breast image.

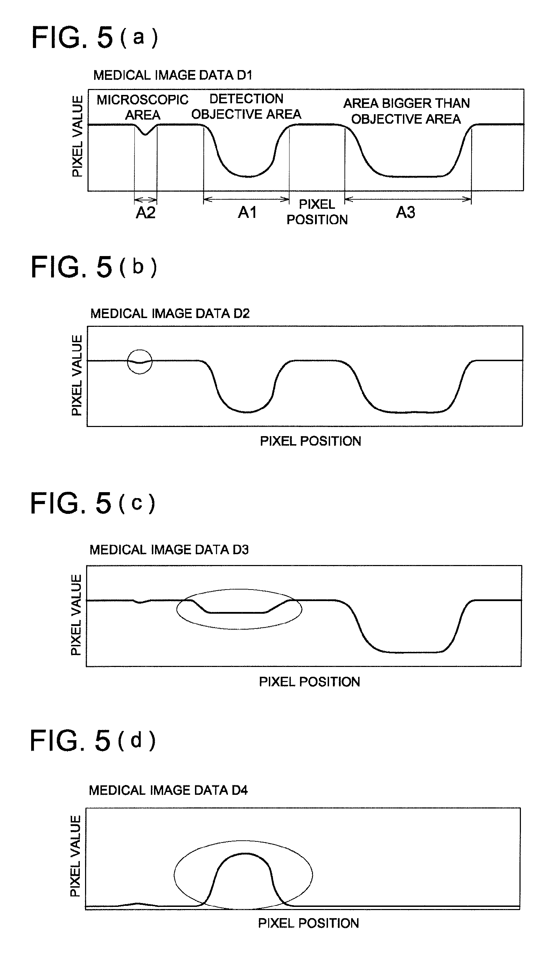

Now, referring to Fig. 4 and Figs. 5(a) - 5(d), the area extraction processing will be detailed in the following.

Fig. 4 shows a flowchart indicting the area extraction processing to be conducted in Step S2 by the CPU 21. Further, Figs. 5(a) - 5(d) show graphs schematically indicating an image before applying the area extraction processing shown in Fig. 4 and processing results of consecutive steps for applying the area extraction processing to the image concerned. In the graph shown in each of Figs. 5(a) - 5(d), the horizontal axis represents positions of the pixels on a certain one line (the same line is indicated in Fig. 5(a) through Fig. 5(d)) of the medical image data (medical image data D1 - medical image data D4), while the vertical axis represents the pixel value (density value).

In the area extraction processing shown in Fig. 4, initially, a size-compression processing is applied to the medical image data D, so as to generate medical image data D1, a sampling pitch of which is about 1.6 mm (Step S11). For instance, if the sampling pitch of original medical image data D is set at 100 $\mu$m, its vertical and horizontal sizes are reduced to 1/16 of the original sizes, respectively. Any kind of size-compression processing algorism, such as averaging pixel values of the pixels in the vicinity of the attention pixel, thinning at constant intervals, etc., can be employed for this purpose. Namely, by reducing the size of the medical image data D in this step, it becomes possible to shorten the processing time to be required for the later processing.

As shown in Fig. 5(a), the breast image includes: a first area (detection objective area) A1, which has a dimension of the abnormal shadow candidate area to be detected (dimension equivalent to that of the abnormal shadow candidate area to be detected) and has a density being lower than that of the surrounding area; a second area (microscopic area) A2, which has a dimension being smaller than that of the abnormal shadow candidate area to be detected and has a density being lower than that of the surrounding area; and a third area (area bigger than objective area) A3, which has a dimension being greater than that of the abnormal shadow candidate area to be detected and has a density being lower than that of the surrounding area. In the processing steps after the area extraction processing, the low density area, having the dimension equivalent to that of the abnormal shadow candidate area to be detected on the size-compressed medical image data D1, is extracted as the detection objective area. In this connection, the later processing will be detailed in the following, by exemplifying such the case that a tumor shadow candidate having a dimension in a range of 5 - 15 mm is to be detected.

When the medical image data D1 are generated by applying the size-compression processing to the medical image data D, a first smoothing processing is applied to the size-compressed medical image data D1, so as to generate medical image data D2 (Step S12).

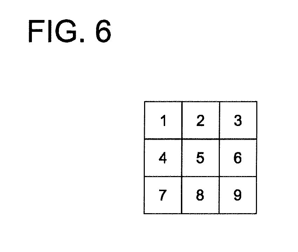

In the first smoothing processing, a first smoothing filter having a mask size of 3 pixels x 3 pixels (refer to Fig. 6) is applied to the medical image data D1, to generate the medical image data D2. The first smoothing filter is a median filter in which a square area (mask) is established by putting a "pixel of interest" (hereinafter, referred to as an attention pixel, for simplicity) of the medical image data D1 at the center of the square area, and aligning the pixel values residing within the mask in a large-to-small order, so as to set its center pixel value as the pixel value of the area 5. By sequentially shifting the position of the mask one pixel to next pixel, the abovementioned operation is repeated for every pixel until the first smoothing processing for all of the pixels included in the medical image data D1 are completed. As a result, as shown in Fig. 5(b), the microscopic area whose horizontal and vertical widths are about 4.8 mm (1.6mm x 3 pixel), respectively, is smoothed.

Successively, a second smoothing processing is applied to the medical image data D2, so as to generate the medical image data D3 (Step S13).

In the second smoothing processing, a second smoothing filter having a mask size of 7 pixels x 7 pixels is applied to the medical image data D2, to generate the medical image data D3.

The second smoothing filter includes a maximum value filter in which a maximum value among the pixel values within the mask size is established as the value of the attention pixel to be located at the center of the mask, and a minimum value filter in which a minimum value among the pixel values within the mask size is established as the value of the attention pixel to be located at the center of the mask, so as to smooth the depression (concaved portion) of the pixel values, dimensions of which are substantially equivalent to those of the mask size, by applying the maximum value filter to the medical image data D2 at first, and then, applying the minimum value filter to the medical image data D2 filtered by the maximum value filter. Generally speaking, since the tumor shadow has such a feature that the level of the X-ray penetration density falls down towards the center of the tumor shadow concerned, it is applicable that the second smoothing filter having dimensions being substantially equivalent to those of the tumor shadow is applied to the tumor shadow concerned, in order to smooth the portion thereof.

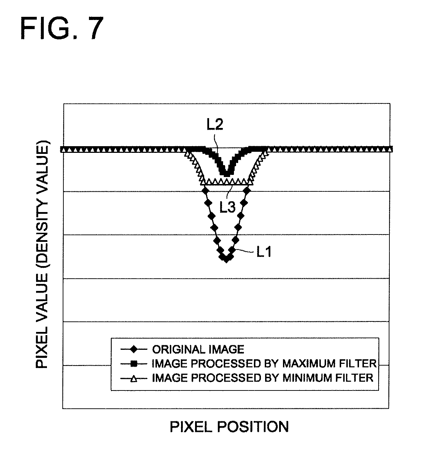

Now, referring to Fig. 7 and exemplifying a one-dimensional data row, the principle of the second smoothing filter will be detailed in the following. In the graph shown in Fig. 7, the horizontal axis represents positions of pixels on the one-dimensional data row, while the vertical axis represents pixel values (density values) on the one-dimensional data row.

A curved line L1 indicates a data low of an original image. The attention pixel is sequentially established from the pixel positioned at the left of the data row of the original image in left-to-right order, so that the maximum value filter, in which the attention pixel is positioned at its center, and which has the mask size of 1 pixel in vertical x 7 pixels in horizontal, is established, and the maximum value among the pixel values within the range of the mask is established as the pixel value of the attention pixel. By shifting the abovementioned operation one pixel to next pixel, the data low indicated by a curved line L2 shown in Fig. 7 can be obtained. Inputting the data row indicated by the curved line L2, the attention pixel is sequentially established from the pixel positioned at the left of the data row indicated by the curved line L2 in left-to-right order, so that the minimum value filter, in which the attention pixel is positioned at its center, and which has the mask size of 1 pixel in vertical x 7 pixels in horizontal, is established, and the minimum value among the pixel values within the range of the mask is established as the pixel value of the attention pixel. Accordingly, as indicated by a curved line L3 shown in Fig. 7, it is possible to obtain the filtered data low, in which the depression of the density of the original data row, indicated by the curved line L1, is smoothed.

As described in the above, the low density area, having dimensions being substantially equivalent to those of the abnormal shadow candidate area A1 to be detected, can be smoothed.

When the second smoothing processing is completed, a differential image data creation processing is implemented. Concretely speaking, by calculating differential components between the pixel values represented by the medical image data D3 shown in Fig. 5(c) and those represented by the medical image data D2 shown in Fig. 5(b), both located at the same pixel positions, respectively, the differential medical image data (medical image data D4), shown in Fig. 5 (d), are generated (Step S14). Successively, a threshold processing is applied to the medical image data D4 by employing a threshold value established in advance, so as to extract only such specific data that have pixel values exceeding the threshold value (Step S15). Accordingly, the specific data, generated in the above, serve as medical image data D5, representing the low density area having dimensions equivalent to those of the abnormal shadow candidate area to be detected.

In this connection, in the area extraction processing abovementioned, the lower limit size of the detection objective area to be extracted (first abnormal shadow size) is determined according to the mask size and the sampling pitch of the first smoothing filter, while the upper limit size of the detection objective area to be extracted (second abnormal shadow size) is determined according to the mask size and the sampling pitch of the second smoothing filter. In other words, by changing and establishing the mask size of the first smoothing filter to be employed for the first smoothing processing conducted in Step S12 shown in Fig. 4, and the mask size of the second smoothing filter to be employed for the second smoothing processing conducted in Step S13 shown in Fig. 4, in accordance with the first abnormal shadow size and the second abnormal shadow size, it is possible to change the dimensions of the area, being the detecting object of the abnormal shadow candidate, to desired dimensions.

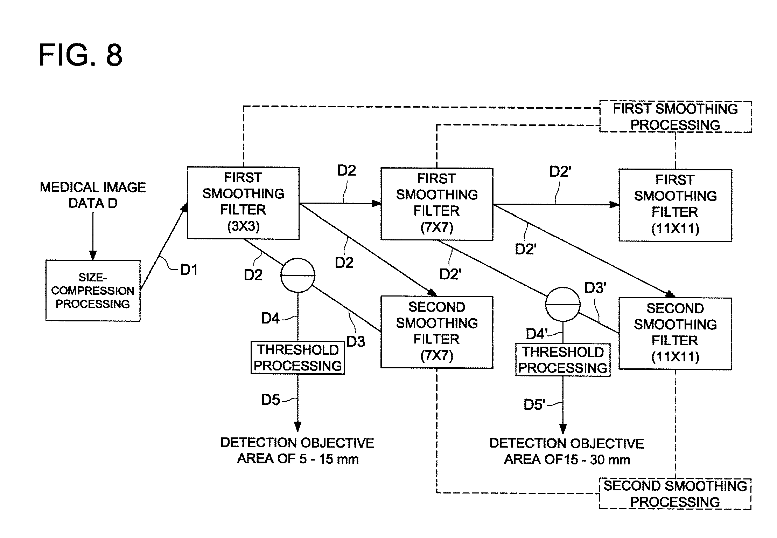

Further, as shown in Fig. 8, by repeatedly conducting the area extraction processing for plural times, while changing and establishing the mask size of the first smoothing filter to be employed for the first smoothing processing conducted in Step S12 shown in Fig. 4, and the mask size of the second smoothing filter to be employed for the second smoothing processing conducted in Step S13 shown in Fig. 4, it becomes possible to extract a plurality of detection objective areas, while classifying them in various sizes. For instance, at first, by employing the first smoothing filter having a mask size of 3 pixels x 3 pixels, the medical image data D2 are generated in Step S12 shown in Fig. 4, and then, by applying the second smoothing filter having a mask size of 7 pixels x 7 pixels to the medical image data D2, the medical image data D3 are generated in Step S13 shown in Fig. 4, and finally, by calculating the differential components between the medical image data D3 and the medical image data D2, concerned, and conducting the threshold processing, detection objective areas substantially in a range of 5 - 15 mm can be extracted. Successively, by applying the first smoothing filter having a mask size of 7 pixels x 7 pixels to the medical image data D2, the medical image data D2' are generated, and then, by applying the second smoothing filter having a mask size of 11 pixels x 11 pixels to the medical image data D2', the medical image data D3' are generated, and finally, by calculating the differential components between the medical image data D3' and the medical image data D2', concerned, and conducting the threshold processing, detection objective areas substantially in a range of 15 - 30 mm can be extracted. As described in the above, it becomes possible to extract the detection objective areas substantially in a range of 5 - 15 mm and the other detection objective areas substantially in a range of 15 - 30 mm, respectively.

Since the tumor shadow is formed in substantially a circular shape, the same processing is applicable for both horizontal and vertical directions of the medical image data. Further, since it is easy to correlate the extracted areas with the tumor shadows, it is specifically preferable to apply the area extraction processing mentioned in the above.

Now, returning to the flowchart shown in Fig. 3, when the area extraction processing is completed, the detection processing of the abnormal shadow candidate is implemented with respect to the area represented by the medical image data D5 extracted from the medical image data D (Step S3).

In the abovementioned detection processing, the tumor shadow candidate is detected by using the curvature filter. In the following descriptions, a method for calculating a characteristic amount by using the curvature filter will be detailed at first, and then, a flow of the abnormal shadow candidate detection processing employing the above method will be detailed later.

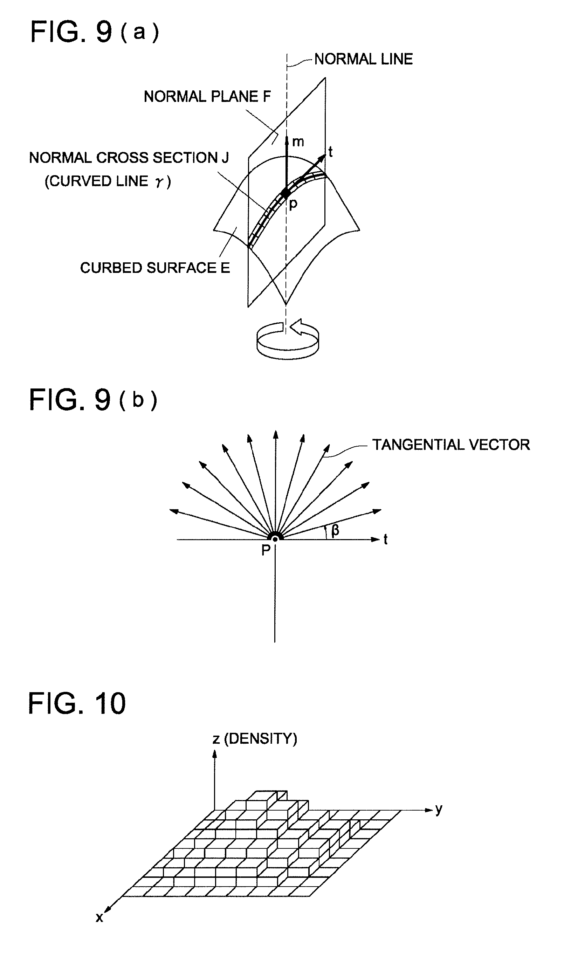

Fig. 9(a) shows positions of pixels and pixel values of the pixels, represented on a two-dimensional coordinates, namely, a curbed surface E representing the density distribution of the breast image composed of signal components in three directions of the density value. In Fig. 9(a), a pixel arbitrarily selected from the pixels residing on the curbed surface E is established as an attention pixel "p", a plane extended by a tangential vector "t" and a normal vector "m" at the attention pixel "p" is defined as a normal plane F and a nodal line between the normal plane F and the curbed surface E (namely, the curbed surface E cut out by the normal plane F) is defined as a normal cross section J.

In this connection, although the curbed surface E is indicated as a smoothly and continuously curbed surface in Fig. 9(a) for the convenience of the explanation, since the breast image is handled as the digital image in reality, the real curbed surface E is represented by the discrete density values (pixel values) constructed stepwise, as shown in Fig 10.

In order to calculate the curvature at the attention pixel "p", the normal plane F (or the tangential vector "t") is made to incrementally rotate for every predetermined angle $\beta$ around the normal vector "m" at attention pixel "p", serving as a rotational axis, as shown in Fig. 9(b), so as to calculate a normal curvature of a curved line $\gamma$ represented by the normal cross section J at attention pixel "p", for every rotating angle. In this connection, the predetermined angle P is determined on the basis of the processing velocity of the image processing apparatus 2. For instance, the predetermined angle $\beta$ can be set at $\pi/2$, $\pi/8$, etc. By increasing the predetermined angle $\beta$, it becomes possible to shorten the time for the arithmetic calculation processing.

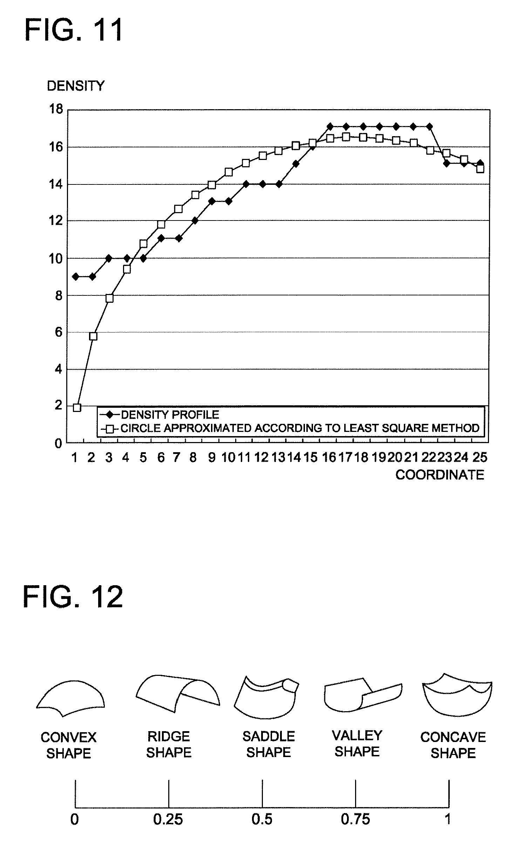

The normal curvature at the attention pixel "p" on the normal cross section J can be found by conducting the steps of: calculating a function for approximately representing the curved line $\gamma$ represented by the normal cross section J according to the least square method; and finding a curvature at the attention pixel "p" represented by the function calculated in the above. In the present embodiment, initially, such the case that the curved line $\gamma$ represented by the normal cross section J (namely, the density profile shown in Fig. 11) is made to approximate to a circular curve, as shown in Fig. 11, will be indicated. The processing for calculating an approximate circle of the curved line $\gamma$ (namely, a circular approximate to the curved line $\gamma$) by employing the least square method, will be detailed in the following. When the center coordinate and the radius of the approximate circle are set at (a, b) and "r", respectively, by employing the two-dimensional coordinate, the approximate circular is expressed as Equation (1) indicated as follow.

$$(X - a)^2 + (Y - b)^2 = r^2 \quad \text{or}$$
$$X^2 + Y^2 - (2Xa + 2Yb) + a^2 + b^2 = r^2 \qquad (1)$$

Successively, establishing each of "n" pixel signal values in the vicinity of the attention pixel "p" residing on the curved line $\gamma$ (an image area within a predetermined range) as $(X_i, Y_i)$ (i = 1, ----, n), a n-dimensional vector A, a matrix B having "n" lines and three columns, and a three-dimensional vector C are defined by Equation (2) indicated as follow.

$$A = \begin{pmatrix} X_1^2 + Y_1^2 \\ X_2^2 + Y_2^2 \\ \vdots \\ X_n^2 + Y_n^2 \end{pmatrix}, \qquad B = \begin{pmatrix} 2X_1 & 2Y_1 & 1 \\ 2X_2 & 2Y_2 & 1 \\ \vdots & \vdots & \vdots \\ 2X_n & 2Y_n & 1 \end{pmatrix}, \qquad C = \begin{pmatrix} a \\ b \\ r^2 - a^2 - b^2 \end{pmatrix} \qquad (2)$$

In order to approximate the curved line $\gamma$ to the Equation (1), it is necessary to find "C", which makes "L" = |A - BC|² minimum. In other words, it may find such a "C" that is employed in the partial differentiation of "L" so as to make "L" zero. Namely, the "C" that fulfills the equation of $\partial L/\partial C = 0$, is expressed by Equation (3) indicated as follow.

$$C = (B^T B)^{-1} \times BA \qquad (3)$$

In Equation (3), the "T" represents a transpose of a matrix, while the $(B^T B)^{-1}$ is a quasi-inverse matrix of "B".

The curved line $\gamma$, represented by the normal cross section J, is approximated by the circle determined by the "C", which fulfills Equation (3). The inverse number of the radius of the approximate circle, determined in the above, is defined as the normal curvature at attention pixel "p" on the curved line $\gamma$. Concretely speaking, when the radius of the approximate circle at the rotational angle $\theta$ is represented by $r(\theta)$, the normal curvature k ($\theta$) at the rotational angle $\theta$ is

expressed by Equation (4) indicated as follow.

$$k(\theta) = 1 / r(\theta) \qquad (4)$$

In this connection, when the center coordinate (a, b) of the approximate circle is positioned at an upper section of the curbed surface E, the sign of the right side of Equation (4) is negative, while, when the center coordinate (a, b) of the approximate circle is positioned at an lower section of the curbed surface E, the sign of the right side of Equation (4) is negative,

Further, it is also possible that a circle determined by three points is fitted to the curved line γ, so as to calculate the normal curvature from the radius of the circle concerned. In this case, the curvature can be calculated according to the steps of: determining a candidate point on the curved line γ and arbitral two points on the curved surface; depicting a circle passing through the above-determined three points; shifting the arbitral two points so as to change the size of the circle concerned; calculating the normal curvature from a radius at which the size change of the circle reaches a climax point.

Other than the circle, each of various kinds of functions, such as an ellipse, a Gaussian function, a quadratic function, etc., can be cited as the approximate function of the curved line γ, being applicable for the above purpose. When employing the ellipse as the approximate function, the inverse numeral of the radius of the ellipse (semi-major axis or semi-minor axis) is defined as the curvature (normal curvature). Further, when employing a Gaussian function Y, indicated by Equation (5) shown as follow, as the approximate function, the value derived from Equation (5) is defined as the curvature (normal curvature).

$$Y = \frac{1}{N} \exp\left( -\frac{X^2}{2\sigma^2} \right) \qquad (5)$$

Still further, the calculating method, to be employed at the time when employing the quadratic function as the approximate function, will be detailed. It is assumed that, in the local coordinate system, the curbed surface E shown in Fig. 9(a) is expressed by Equation (6) indicated as follow.

$$Z(x, y) = \frac{1}{2}\left( ax^2 + 2bxy + cy^2 \right) + O(x, y)^k \qquad (6)$$

where "a", "b" and "c" are constant values, and
$O(x, y)k$ represents a third term or a higher order term.

In this case, the normal curvature k(θ) at the attention pixel "p", which resides on the normal cross section J (curved line γ) inclined at an angle θ in respect to the x-axis, is expressed by Equation (7) indicated as follow.

$$k(\theta) = a\cos^2\theta + 2b\cos\theta\sin\theta + c\sin^2\theta \qquad (7)$$

Herein, a quadratic function, which serves as an approximate function of the curved line γ, is assumed on the basis of the Equation (6) representing the curbed surface E. Then, the normal curvature is found by calculating a coefficient of the second term, a coefficient of the first term and a constant term, which are belong to the above-assumed quadratic function, according to the least square method.

Initially, it is assumed that the approximate function of the curved line γ is expressed on the two-dimensional coordinate by the quadratic function of Equation (8) indicated as follow.

$$Y = a'X^2 + b'X + c' \qquad (8)$$

Successively, "n" pieces of image signal values in the vicinity of the attention pixel "p" on the curved line $\gamma$ (image area within a predetermined range) are defined as $(X_i, Y_i)$ (i = 1, ----, n), so as to calculate an average square difference value S between value of $a'X_i^2 + b'X_i + c'$ and $Y_i$ being output value of this quadratic function. Still successively, the coefficients a', b', c' are calculated by setting the value, derived by applying partial differentiation to the average square difference value S in respect to the coefficients a', b', c', to zero, so as to determine the quadratic function that serves as the approximate function of the curved line $\gamma$.

By substituting the coefficients a', b', c' in the Equation (8), which are derived by employing the least square method, for coefficients a, b, c of the Equation (7), the normal curvature k ($\theta$) can be calculated. In this connection, even when a multi-dimensional polynomial function (high-order polynomial function) equal to or higher than trinomial is employed as the approximate function, it is also possible to calculate the normal curvature in the method same as that employed in the above case of the quadratic function, and further, it is possible to acquire information of curved shape more finer than that represented by the quadratic function.

When the normal curvature is calculated from the approximate function of the curved line $\gamma$, according to the procedure described in the foregoing, a "Shape Index" can be calculated as follow. Since the shape of the normal cross section J (curved line $\gamma$) changes according as the normal plane F rotates around the normal line of the attention pixel "p", serving as the rotational axis, the value of normal curvature k ($\theta$) also changes corresponding to the rotated angle $\theta$. In other words, both the maximum value and the minimum value of the normal curvature k ($\theta$) emerge at certain rotated angles $\theta$ of the normal plane F. Under the condition that the maximum value and the minimum value among values of the normal curvature k ($\theta$) calculated at rotated angles $\theta$ in a range of 0 - $\pi$ ($0 \leq \theta < \pi$) are established as $k_{max}$ and $k_{min}$, respectively, a Shape Index SI is defined by Equation (9) indicated as follow.

$$SI = \frac{1}{2} - \frac{1}{\pi} \arctan \frac{k_{max} + k_{min}}{k_{max} - k_{min}} \qquad (9)$$

The Shape Index SI derived from Equation (9) represents the curved shape of the curbed surface E in the image area within the predetermined range centering the attention pixel "p".

Fig. 12 shows various kinds of curved shapes corresponding to the values of the Shape Index SI. At a whitish portion (namely, a low density portion) of the processing objective image, the value of Shape Index SI approaches 1 and the curved shape becomes concave. On the other hand, at a darkish portion (namely, a high density portion) of the processing objective image, the value of Shape Index SI approaches zero and the curved shape becomes convex.

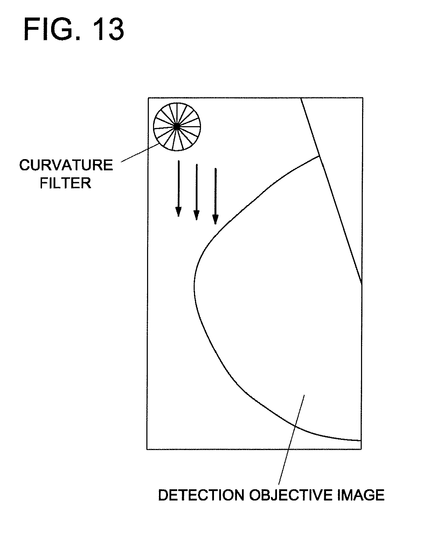

Fig. 13 shows a curvature filter and the processing objective image employed in the present embodiment. As shown in Fig. 13, by scanning the curvature filter all over the extracted area extracted in Step S3 shown in Fig. 3, each of Shape Index values corresponding to each of the pixels is calculated, so as to create the Shape Index image in which concave and convex portions of the density distribution are emphasized. Provided that the range of Shape Index values to be found corresponding to the kind of abnormal shadow is established in advance, by detecting a signal area, in which the Shape Index value resides in a specific range, from the Shape Index image, it is possible to detect the abnormal shadow concerned. For instance, a tumor tends to be formed in a concaved shape exhibiting a smoothly changing Gaussian distribution. Accordingly, it becomes possible to detect a candidate area of the tumor shadow, by detecting the image area at which the Shape Index value is in a range of 0.75 - 1.00. In this connection, it is also applicable that the processing objective area is established at first, so as to scan the curvature filter over the pixels on the processing objective area established in the above.

Further, although the curved shape of the density distribution at the time of outputting an image onto the film has been described in the present embodiment, it is also applicable that a curved shape of a luminance distribution at the time of outputting an image onto the displaying monitor is handled. Concretely speaking, in the case of luminance, a high density value corresponds to the whitish area, while a low density value corresponds to the darkish area. Accordingly, in the case of luminance distribution, according as the value of Shape Index SI approaches 1, the curved shape becomes convex, while, according as the value of Shape Index SI approaches zero, the curved shape becomes concave.

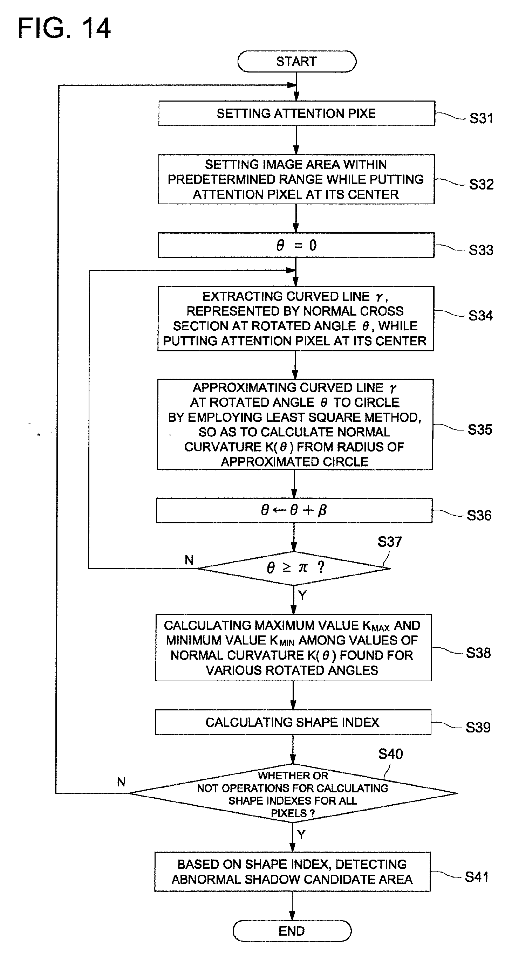

[0074], [0075], [0076], [0077], [0078], [0079], Next, referring to Fig. 14, the detection processing of the abnormal shadow candidate, employing the curvature filter mentioned in the above, will be detailed in the following.

The detection processing of the abnormal shadow candidate indicated in the flowchart, shown in Fig. 14, includes the steps of: setting the attention pixel "p" with respect to the extracted area, extracted from the medical image data D in Step S2 shown in Fig. 3, (Step S31); setting the image area within the predetermined range in which the attention pixel "p" is disposed at its center (Step S32); setting the rotated angle $\theta$ around the normal line of the attention pixel "p", serving as the rotation axis, at zero (Step S33); extracting the curved line $\gamma$, represented by the normal cross section J at the rotated angle $\theta$, by cutting out the curbed surface E, indicating the density distribution at the image area set in Step S32, with the normal plane F at the rotated angle $\theta$ (Step S34), so as to store the pixel signal values (density values) on the curved line γ into the storage section 25; approximating the curved line γ at the rotated angle θ to a circle by employing the least square method, so as to calculate the normal curvature $k(\theta)$ at rotated angle θ of the attention pixel "p" from the radius of the approximated circle (Step S35); setting the value derived by adding the P to the rotated angle θ as a current rotated angle θ (Step S36); determining whether or not the current rotated angle θ is equal to or greater than π (Step S37); returning to Step S34, so as to repeat the processing from Step S34 to Step S37 with respect to the rotated angle θ concerned, when determining that the current rotated angle θ is smaller than π (Step S37: No); calculating the maximum value $k_{max}$ and the minimum value $k_{min}$ among the values of the normal curvature $k(\theta)$ found for various rotated angles (Step S38), when determining that the current rotated angle θ is equal to or greater than π (Step S37: Yes); calculating the Shape Index SI by substituting the maximum value $k_{max}$ and the minimum value $k_{min}$ calculated in Step S38 for those of Equation (9) (Step S39), so as to store the calculated value of the Shape Index SI into the characteristic amount file, provided in the storage section 25, as the characteristic amount of the current attention pixel "p"; determining whether or not the operations for calculating Shape Indexes for all pixels residing within the extracted area are completed (Step S40); returning to Step S31, so as to set a next attention pixel within the extracted area (Step S31), and to repeat the processing from Step S32 to Step S39 with respect to the next attention pixel set in Step S31, when determining that the operations for calculating Shape Indexes for all pixels residing within the extracted area are not yet completed (Step S40: No); creating the Shape Index image in which concave and convex portions of the density distribution are emphasized, based on the Shape Indexes of all pixels calculated by scanning the curvature filter all over the pixels concerned, when determining that the operations for calculating Shape Indexes for all pixels residing within the extracted area are completed (Step S40: Yes); and detecting an image area residing within a range, in which the value of the Shape Index SI corresponds to a specific abnormal shadow, as the abnormal shadow candidate concerned (Step S41).

Since it is assumed that the tumor is the detecting object in the abovementioned flowchart, for instance, by detecting the image area in which the values of the Shape Index SI are in a range of 0.75 - 1.00, the tumor shadow candidate area can be recognized.

After detecting the abnormal shadow candidate area, the processing shown in Fig. 3 enters into Step S4, so as to display the detecting result of the abnormal shadow candidate on the display section 23 (Step S4). For instance, the breast image based on the medical image data D is displayed on the display section 23 in such a manner that the candidate area detected as the abnormal shadow candidate is designated by a specific arrow symbol, or by a specific color or the like, in order to discriminate the candidate area from other areas. Further, it is also applicable to output the characteristic amount at the abnormal shadow candidate concerned.

As described in the foregoing, according to the present embodiment, as the pre-processing of the detection processing of the abnormal shadow candidate, employing the curvature filter, the detection processing objective area is extracted by using the first and second smoothing filters. Accordingly, it becomes possible to apply the detection processing employing the curvature filter only to the extracted area, resulting in a drastic reduction of the processing time. Further, since the detection processing employing the curvature filter can sensitively detect the image pattern inherent to the tumor, the detection objective areas are limited to those having a certain predetermined size in advance before applying the above detection processing. Therefore, since it becomes possible to exclude in advance such an area that has a size to be regarded as a false positive, judging from expected dimensions of the corresponding tumor concerned, from the detection object, it becomes possible to reduce the number of false positive candidates. In other words, when applying such the detection processing as the curvature filter that has a high detection sensitivity, by conducting the area extraction processing using the smoothing filter preceding to the detection processing, it becomes possible to obtain a synergistic effect combining the reduction of the processing time and the improvement of the detecting accuracy with each other.

Further, since the mask size of each of the first and second smoothing filters is changeable, it is possible to extract an appropriate area corresponding to the size of the lesion species to be established as the detecting object. Although it is possible to detect the abnormal shadow candidate while taking its size into account by changing the mask size to which the curvature filter is to be applied, the time consumption required for the detection processing conducted by using the curvature filter tends to be greater than that required for the extraction processing conducted by using the smoothing filter. To overcome the above drawback, by employing the smoothing filter to extract an area, which has the size to be established as the detecting object, in advance, and employing the curvature filter so as to conduct the detection processing within a range of the extract area having the abovementioned size, it becomes possible not only to reduce the processing time, but also to conduct the detection processing while taking the size of candidate area into account, resulting in an improvement of the detecting accuracy.

Further, by changing and setting the mask size of the filter to be employed for each of the first and second smoothing processing, it becomes possible to change the size of the detection objective area to be extracted, corresponding to the dimensions of the abnormal shadow species to be detected. Accordingly, by extracting only such the area that has a size, which is limited to that inherent to the abnormal shadow species being a detecting object, in the stage of pre-processing, it becomes possible not only to reduce the processing time in the stage of post-processing, but also to remove the false positive candidate.

Still further, since it becomes possible to extract the detection objective areas while classifying them into various sizes of the abnormal shadow candidates to be detected, by repeating the processing from Step S12 to Step S15, shown in Fig. 4, while changing the mask size, it becomes possible to conduct an operation for detecting the abnormal shadow candidate according to a plurality of detecting models.

In this connection, from the detectability viewpoint, it is preferable that the pixel size of the curvature filter to be used for the extracted area is set at: 15 x 15 pixel size (6 x 6 mm), when an image of sampling pitch 400 μm is employed, and a tumor shadow having an extracted area in a range of 5 - 15 mm is established as the detecting object; or 35 x 35 pixel size (14 x 14 mm), when an image of sampling pitch 400 μm is employed, and a tumor shadow having an extracted area in a range of 15 - 30 mm is established as the detecting object.

Yet further, although the image processing apparatus 2 conducts the first and second smoothing processing, the operation for calculating the characteristic amount, the detection processing of the abnormal shadow candidate, etc., embodied in the present invention, in the present embodiment mentioned in the foregoing, the scope of the present invention is not limited to the abovementioned embodiment. It is also applicable that the abovementioned processing are conducted in the other apparatus (such as a server, etc.) coupled to the medical imaging system 100, or conducted in an independent apparatus newly installed in the medical imaging system 100.

**Claims**

1. An abnormal shadow candidate detecting method, **characterized by** comprising:

   a first processing process for applying a first smoothing filter processing to medical image inputted;
   a second processing process for applying a second smoothing filter processing to processed image to which the first smoothing filter processing is applied;
   an extraction processing process for extracting a specific area, from which an abnormal shadow candidate is to be detected, from processed image to which the second smoothing filter processing is applied;
   a characteristic amount calculating process for calculating a characteristic amount representing a curved shape indicating a density distribution of the specific area extracted; and
   an abnormal shadow candidate detecting process for conducting a detection processing of the abnormal shadow candidate, based on the characteristic amount calculated.

2. The abnormal shadow candidate detecting method, recited in claim 1, **characterized in that**
   the method further comprises a process for reducing the medical image inputted; and
   the first smoothing filter processing is applied to the reduced medical image inputted in the first processing process.

3. The abnormal shadow candidate detecting method, recited in claim 1 or 2, **characterized in that**
   a Shape Index is calculated as the characteristic amount, representing the curved surface indicating the density distribution, in the characteristic amount calculating process.

4. The abnormal shadow candidate detecting method, recited in any one of claims 1 - 3, **characterized in that**
   an abnormal shadow species to be established as an detection object in the abnormal shadow candidate detecting process is a tumor.

5. An abnormal shadow candidate detecting method, **characterized by** comprising:

   a process for setting a first smoothing filter corresponding to a first abnormal shadow size to be extracted;
   a process for setting a second smoothing filter corresponding to a second abnormal shadow size to be extracted;
   an extracting process for extracting a specific area having a desired dimension to detect an abnormal shadow candidate, by applying the first smoothing filter and the second smoothing filter to medical image inputted;
   a characteristic amount calculating process for calculating a characteristic amount representing a curved shape indicating a density distribution of the specific area; and
   an abnormal shadow candidate detecting process for conducting a detection processing of the abnormal shadow candidate, based on the characteristic amount calculated.

6. An abnormal shadow candidate detecting apparatus, **characterized by** comprising:

   a first smoothing processing means for applying a first smoothing filter processing to medical image inputted;

a second smoothing processing means for applying a second smoothing filter processing to processed image to which the first smoothing filter processing is applied;

an extracting means for extracting a specific area, from which an abnormal shadow candidate is to be detected, from processed image to which the second smoothing filter processing is applied;

a characteristic amount calculating means for calculating a characteristic amount representing a curved shape indicating a density distribution of the specific area extracted; and

an abnormal shadow candidate detecting means for conducting a detection processing of the abnormal shadow candidate, based on the characteristic amount calculated.

7. The abnormal shadow candidate detecting apparatus, recited in claim 6, **characterized in that**
the apparatus further comprises a reduction processing means for reducing the medical image inputted; and
the first smoothing processing means applies the first smoothing filter processing to the reduced medical image inputted.

8. The abnormal shadow candidate detecting apparatus, recited in claim 6 or 7, **characterized in that**
the characteristic amount calculating means calculates a Shape Index as the characteristic amount, representing the curved surface indicating the density distribution

9. The abnormal shadow candidate detecting method, recited in any ane of claims 6 - 8, **characterized in that**
an abnormal shadow species to be established as an detection object in the abnormal shadow candidate detecting process is a tumor.

10. An abnormal shadow candidate detecting apparatus, **characterized by** comprising:

a first setting means for setting a first smoothing filter corresponding to a first abnormal shadow size to be extracted;

a second setting means for setting a second smoothing filter corresponding to a second abnormal shadow size to be extracted;

an extracting means for extracting a specific area having a desired dimension to detect an abnormal shadow candidate, by applying the first smoothing filter and the second smoothing filter to medical image inputted;

a characteristic amount calculating means for calculating a characteristic amount representing a curved shape indicating a density distribution of the specific area; and

an abnormal shadow candidate detecting means for conducting a detection processing of the abnormal shadow candidate, based on the characteristic amount calculated.

# FIG. 1

100

| IMAGE DATA CREATING APPARATUS | | IMAGE PROCESSING APPARATUS |
|---|---|---|

1

N

# FIG. 2

2

CPU — 21

22 — OPERATING SECTION

STORAGE SECTION — 25

23 — DISPLAY SECTION

COMMUNICATION CONTROLLING SECTION — 26

24 — RAM

27

# FIG. 3

```
        START
          │
          ▼
┌─────────────────────┐
│ INPUTTING IMAGE DATA │ ── S1
└─────────────────────┘
          │
          ▼
┌─────────────────────┐
│  AREA EXTRACTION     │ ── S2
│   PROCESSING         │
└─────────────────────┘
          │
          ▼
┌─────────────────────┐
│  ABNORMAL SHADOW     │
│ CANDIDATE DETECTION  │ ── S3
│   PROCESSING         │
└─────────────────────┘
          │
          ▼
┌─────────────────────┐
│ DISPLAYING DETECTION │ ── S4
│     RESULT           │
└─────────────────────┘
          │
          ▼
         END
```

# FIG. 4

```
   AREA EXTRACTION
     PROCESSING
          │
          ▼
┌─────────────────────┐
│  SIZE-COMPRESSION    │ ── S11
│   PROCESSING         │
└─────────────────────┘
          │
          ▼
┌─────────────────────┐
│  FIRST SMOOTHING     │ ── S12
│   PROCESSING         │
└─────────────────────┘
          │
          ▼
┌─────────────────────┐
│  SECOND SMOOTHING    │ ── S13
│   PROCESSING         │
└─────────────────────┘
          │
          ▼
┌─────────────────────┐
│ DIFFERENTIAL IMAGE DATA │ ── S14
│  CREATION PROCESSING │
└─────────────────────┘
          │
          ▼
┌─────────────────────┐
│ THRESHOLD PROCESSING │ ── S15
└─────────────────────┘
          │
          ▼
         END
```

# FIG. 5(a)

MEDICAL IMAGE DATA D1

PIXEL VALUE

MICROSCOPIC AREA    DETECTION OBJECTIVE AREA    AREA BIGGER THAN OBJECTIVE AREA

A2        A1        PIXEL POSITION        A3

# FIG. 5(b)

MEDICAL IMAGE DATA D2

PIXEL VALUE

PIXEL POSITION

# FIG. 5(c)

MEDICAL IMAGE DATA D3

PIXEL VALUE

PIXEL POSITION

# FIG. 5(d)

MEDICAL IMAGE DATA D4

PIXEL VALUE

PIXEL POSITION

FIG. 6

| 1 | 2 | 3 |
|---|---|---|
| 4 | 5 | 6 |
| 7 | 8 | 9 |

# FIG. 7

# FIG. 8

MEDICAL IMAGE DATA D → SIZE-COMPRESSION PROCESSING

D1 → FIRST SMOOTHING FILTER (3X3)

FIRST SMOOTHING PROCESSING

D2 → FIRST SMOOTHING FILTER (7X7) → D2' → FIRST SMOOTHING FILTER (11X11)

D2 → SECOND SMOOTHING FILTER (7X7) → D3 → THRESHOLD PROCESSING → D5 → DETECTION OBJECTIVE AREA OF 5 - 15 mm

D2, D4

D2' → SECOND SMOOTHING FILTER (11X11) → D3' → THRESHOLD PROCESSING → D5' → DETECTION OBJECTIVE AREA OF 15 - 30 mm

D4'

SECOND SMOOTHING PROCESSING

20

## FIG. 9 ( a )

NORMAL LINE

NORMAL PLANE F

NORMAL CROSS SECTION J
(CURVED LINE $\gamma$)

CURBED SURFACE E

## FIG. 9 ( b )

TANGENTIAL VECTOR

## FIG. 10

z (DENSITY)

y

x

# FIG. 11

DENSITY

COORDINATE

# FIG. 12

| CONVEX SHAPE | RIDGE SHAPE | SADDLE SHAPE | VALLEY SHAPE | CONCAVE SHAPE |

0        0.25        0.5        0.75        1

# FIG. 13

CURVATURE
FILTER

DETECTION OBJECTIVE IMAGE

# FIG. 14

START

SETTING ATTENTION PIXE — S31

SETTING IMAGE AREA WITHIN PREDETERMINED RANGE WHILE PUTTING ATTENTION PIXEL AT ITS CENTER — S32

$\theta = 0$ — S33

EXTRACTING CURVED LINE $\gamma$, REPRESENTED BY NORMAL CROSS SECTION AT ROTATED ANGLE $\theta$, WHILE PUTTING ATTENTION PIXEL AT ITS CENTER — S34

APPROXIMATING CURVED LINE $\gamma$ AT ROTATED ANGLE $\theta$ TO CIRCLE BY EMPLOYING LEAST SQUARE METHOD, SO AS TO CALCULATE NORMAL CURVATURE K($\theta$) FROM RADIUS OF APPROXIMATED CIRCLE — S35

$\theta \leftarrow \theta + \beta$ — S36

S37

N    $\theta \geq \pi$ ?

Y

CALCULATING MAXIMUM VALUE $K_{MAX}$ AND MINIMUM VALUE $K_{MIN}$ AMONG VALUES OF NORMAL CURVATURE K($\theta$) FOUND FOR VARIOUS ROTATED ANGLES — S38

CALCULATING SHAPE INDEX — S39

S40

N    WHETHER OR NOT OPERATIONS FOR CALCULATING SHAPE INDEXES FOR ALL PIXELS ?

Y

BASED ON SHAPE INDEX, DETECTING ABNORMAL SHADOW CANDIDATE AREA — S41

END

## INTERNATIONAL SEARCH REPORT

| International application No. |
|---|
| PCT/JP2006/313820 |

**A. CLASSIFICATION OF SUBJECT MATTER**
$A61B6/00$(2006.01)i, $G06T1/00$(2006.01)i, $G06T7/60$(2006.01)i, $A61B5/055$ (2006.01)n

According to International Patent Classification (IPC) or to both national classification and IPC

**B. FIELDS SEARCHED**

Minimum documentation searched (classification system followed by classification symbols)
A61B6/00, G06T1/00, G06T7/60, A61B5/055

Documentation searched other than minimum documentation to the extent that such documents are included in the fields searched
Jitsuyo Shinan Koho          1922-1996     Jitsuyo Shinan Toroku Koho    1996-2006
Kokai Jitsuyo Shinan Koho    1971-2006     Toroku Jitsuyo Shinan Koho    1994-2006

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| Y | JP 2005-80758 A  (Konica Minolta Medical & Graphic, Inc.), 31 March, 2005 (31.03.05), Full text; all drawings & US 2005/0053270 A1 | 1-4,6-9 |
| Y | JP 2002-209881 A  (GE Medical Systems Global Technology Co., LLC), 30 July, 2002 (30.07.02), Figs. 3, 4; Par. Nos. [0026] to [0031] (Family: none) | 1-4,6-9 |
| Y | JP 2002-112985 A  (Konica Corp.), 16 April, 2002 (16.04.02), Par. No. [0022] (Family: none) | 2,7 |

☐ Further documents are listed in the continuation of Box C.          ☐ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered   to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 17 August, 2006 (17.08.06) | 29 August, 2006 (29.08.06) |

| Name and mailing address of the ISA/ | Authorized officer |
|---|---|
| Japanese Patent Office | |
| Facsimile No. | Telephone No. |

Form PCT/ISA/210 (second sheet) (April 2005)

**EP 1 908 404 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2002112986 A
- JP 2001346787 A